# EUROPEAN PATENT APPLICATION

(11) **EP 2 316 433 A1**
(43) Date of publication of application: **04.05.2011**
(21) Application number: 09173727.0
(22) Date of filing: 22.10.2009
(51) Int. Cl.: A61K 9/20, A61K 31/192

(54) **Orally disintegrating tablets of flurbiprofen**

(71) Applicant: Abdi Ibrahim Ilac Sanayi Ve Ticaret Anonim Sirketi, 34555 Hadimkoy, Istanbul (TR)
(72) Inventor: Farshi, Farhad, 34555 ISTANBUL (TR); Avci, Recep, 34555 ISTANBUL (TR); Sahin, Sinem, 34555 ISTANBUL (TR); Koc, Fikret, 34555 ISTANBUL (TR); Soylemez, Serdar, 34555 ISTANBUL (TR)

(57) **Abstract**

This invention is related to orally disintegrating tablets of flurbiprofen characterized in that flurbiprofen particles are coated with a coating agent, a pore former and a surfactant.

## Description

### Technical Field

The present invention is related to orally disintegrating tablets of flurbiprofen characterized in that flurbiprofen particles are coated with a coating agent, a pore former and a surfactant.

### Background Art

The article CIRRI, Marzia, et al. Development of Fast-Dissolving Tablets of Flurbiprofen-Cyclodextrin Complexes. Drug Development and Industrial Pharmacy. 2005, vol.31, no.7, p.697-707. provides fast dissolving tablets as flurbiprofen-cyclodextrin complexes.

### Disclosure of Invention

The present invention provides fast disintegrating or fast dissolving dosage form of flurbiprofen. Fast disintegrating or fast dissolving dosage forms include an effective amount of flurbiprofen or a pharmaceutically acceptable salt thereof and at least one pharmaceutically acceptable excipient.

According to this invention, in fast disintegrating or fast dissolving dosage forms, pharmaceutical formulations are disintegrated in less than three minutes in oral cavity. More preferably, the pharmaceutical formulations are disintegrated in the oral cavity in less than two minutes and most preferably the pharmaceutical formulations are disintegrated in oral cavity in less than one minute.

Fast-dissolving or fast-disintegrating tablets are useful for elderly people, children and patients who have difficulties in swallowing tablets. Such peoples are unwilling and/or unable to swallow tablets, capsules or other traditional solid dosage forms. This is especially the case of pharmaceuticals for paediatric or geriatric use. To solve this problem, tablets that disintegrate rapidly in the mouth were developed. Such tablets are rapidly disintegrated when contact with saliva in the oral cavity.

According to this invention, the pharmaceutical formulations in a dosage form are, but not limited to, rapidly disintegrating tablets, lingual strips, sublingual tablets, lyophilized wafers, granulated particles, sublingual strips, spray and whatsoever.

A fast-disintegrating tablet may have many numerous shapes, such as dish-like, ellipsoid, rods, granules, blocks, cubes with rounded edges, or any other shape suitable for pharmaceutical administration.

Flurbiprofen is administered in pharmaceutically acceptable doses.

According to this invention, fast disintegrating or fast dissolving dosage form may include one or more pharmaceutically acceptable excipients, carriers, or diluents. In fast disintegrating or fast dissolving formulations, surfactants, diluents, sweeteners, disintegrants, binders, lubricants, glidants, colorants, flavors, stabilizing agents, surfactants, mixtures thereof and the like can be used.

Diluents are, but not limited to, mannitol, sorbitol, xylitol, microcrystalline cellulose, silicified microcrystalline cellulose, hydroxypropyl methylcellulose, hydroxypropyl cellulose, pullulan and fast dissolving carbohydrates such as Pharmaburst™, mixtures thereof and the like.

Glidants are, but not limited to, silicon dioxide, colloidal silicon dioxide, calcium silicate, magnesium silicate, magnesium trisilicate, talc, starch, mixtures thereof or whatsoever.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose, tragacanth, sorbitol, acacia, alginates, carrageenan, xanthan gum, locust bean gum and gum arabic, waxes,polyacrylamide, mixtures thereof, and whatsoever.

Lubricants are, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof and the like.

Disintegrants are, but not limited to, sodium starch glycolate, sodium carboxymethyl cellulose, calcium carboxymethyl cellulose, croscarmellose sodium, crospovidone, chitosan, agar, alginic acid, calcium alginate, methyl cellulose, microcrystalline cellulose, powdered cellulose, lower alkylsubstituted hydroxypropyl cellulose, hydroxylpropyl starch, low-substituted hydroxypropylcellulose, polacrilin potassium, starch, pregelatinized starch, sodium alginate, magnesium aluminum silicate, polacrilin potassium, povidone, sodium starch glycolate, mixtures thereof and the like.

Disintegrant are in mixture or in combination with other excipient or excipients. For example Ludiflash® is made up of Kollidon® CL-SF,Kollicoat® SR30D and mannitol. Kollicoat® SR30D is made up Polyvinyl acetate stabilized with polyvinylpyrrolidone and sodium lauryl sulphate. Kollidon® CL-SF is crospovidone. Ludiflash® provides tablets disintegrating in the mouth within seconds.

Flavors are, but not limited to, cinnamon oil, essence of apple, essence of pear, essence of peach, essence of grape, essence of strawberry, essence of raspberry, essence of cherry, essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit, vanilla, benzaldehyde, aldehyde C-8, aldehyde C-9, aldehyde C-12, tolyl aldehyde, mixtures thereof or whatsoever.

Stabilizing agents are, but not limited to, polyvinyl pyrrolidone, polymethacrylate, polyvinyl acetate phthalate, alkyl cellulose, hydroxyl alkyl cellulose, polyethylene glycol, polyethylene castor oil, polyethylene glycol sorbitan fatty acid, polyethylene polypropylene glycol, polyethylene oxide, polyoxyethylene alkyl ether, polyoxyethylene stearate, caproic acid, caprilic acid, capric acid, lauric acid, myhstic acid, palmitic acid, stearic acid, arachidic acid, behenic acid, and lignoceric acid, hydrogenated coconut fatty acids, hydrogenated palm fatty acids, hydrogenated rapeseed fatty acids, hydrogenated tallow fatty acids, and hydrogenated castor oil fatty acids, 2-ethylhexanoic acid, isostearic acid, methanol, ethanol, n-propanol, n-butanol, n-penthanol, lauryl alcohol, myristyl alcohol, palmyl alcohol, stearyl alcohol, behenyl alcohol, isopropanol, isobutanol, sec-butanol, 2-ethylhexanol, isododecyl alcohol, isotridecyl alcohol, isostearyl alcohol, alcohol 2-octyldodecyl, glycolic acid, lactic acid, glyceric acid, tartronic acid, malic acid, and citric acid, ethylenglycol, diethylenglycol, triethylenglycol, propylenglycol, dipropylenglycol, glycerin, diglycehn, triglycerin, sorbitol and sorbitan, sodium palmitate, sodium stearate, potassium stearate, potassium palmitate, and sodium myristate, isopropyl myristate, isopropyl palmitate, isopropyl stearate, n- butyl stearate, 2-ethylexyl palmitate, 2-ethylexyl stearate, 2-octyldodecyl myristate, 2-ethylhexyl isostearate, isostearyl isostearate, isononyl isononanoate, thethyl citrate, butyl citrate, methyl lactate, ethyl lactate, n-butyl lactate, and n-propyl lactate, diethylenglycol monoethyl ether, ethylenglycol monobutyl ether, diethylenglycol monobutyl ether, triethylenglycol monobutyl ether, thethylenglycol monoethyl, ethylenglycol monomethyl ether, diethylenglycol mono-n-propyl ether, 2-octyldodecyl myhstate, isostearyl isostearate, thethyl citrate, triethyl citrate, mixtures thereof and the like.

Sweeteners are but not limited to, corn syrup, dextrose, invert sugar, fructose, saccharin, aspartame, acesulfame-K, Stevia rebaudiana, sucralose, sorbitol, mannitol, zylitol, mixtures thereof and the like.

The fast-disintegrating or fast dissolving dosage form of flurbiprofen is a solid dosage form that does not require water for swallowing.

Fast disintegrating or fast dissolving dosage form is typically orally disintegrating tablet that dissolves or disperses rapidly when in contact with saliva.

The compositions of the present invention can be prepared through using pharmaceutical technologies including, but not limited to, spray drying, freeze-drying, heat molding, tablet molding, sublimation and the like.

Orally disintegrating tablet may comprise sugar-based excipients, super-disintegrating agents, effervescent agents and further suitable excipients.

Another embodiment of the present invention provides a carbohydrate-based fast dissolving dosage form. Co-processed carbohydrates can be used in embodiments.

This invention provides taste masked orally disintegrating tablets.

In fast dissolving or fast disintegrating dosage forms one or more taste masking excipients such as flavors, sweeteners, acidic amino acids, lipids, and surfactants can further be used.

A preferred embodiment is created by sugar-based fast-dissolving method or one or more sugar - based excipients. These excipients are, but not limited to, amylose, dextrose, fructose, sucrose, maltose, erythritol, isomalt, lacitol, maltitol, mannitol, sorbitol, starch hydrolysate, polydextrose, glucose, xylitol, mixtures thereof and the like.

Flurbiprofen orally disintegrating tablet comprises; flurbiprofen is in the range of from 5 % to 50 %, binder is in the range of from 0.1 % to 5 %, coating agent is in the range of from 0.1 % to 5 %, pore former is in the range of from 0.01 % to 5 %, disintegrant is in the range of from 10 % to 90 %, sweetener is in the range of from 0.1 % to 10 %, stabilizing agent is in the range of from 0.1 % to 5 %, flavour is in the range of from 0.1 % to 5 %, glidant is in the range of from 0.1 % to 5 %, lubricant is in the range of from 0.1 % to 20 %, surfactant is in the range of from 0.1 % to 10 %, by weight.

In a preferred embodiment of orally disintegrating tablet comprises flurbiprofen is 25 %, binder is 1.88 %, coating agent 0.50 %, pore former is 0.05 %, disintegrant is 66.29 %, sweetener is 0.75 %, stabilizing agent 0.50 %, flavour is 1.50 %, glidant is 0.50 %, lubricant is 2.50 %, surfactant is 0.53 % by weight.

Compositions may further include remaining excipient or excipients from coating solution in trivial amounts.

An additional problem arises from oral administration. Orally disintegrating tablets contains bitter or irritating drugs need to be masked in the oral cavity. Therefore a taste masking layer should be created.

The taste masking layer comprises one or more coating agents. Non-limiting examples of suitable coating agents include, but not limited to, ethyl cellulose, methyl cellulose, hydroxypropyl-methyl cellulose, hydroxyethyl cellulose, hydroxypropyl cellulose, polyvinylalcohol, polyvinyl acetate, cellulose acetate,cellulose acetate phthalate, methylcellulose phthalate, hydroxymethyl cellulose phthalate, hydroxypropylmethylcellulose phthalate, hydroxypropylmethylcellulose succinate, cellulose acetate butyrate, methacrylate copolymers, polymethacrylates, acetyltributyl citrate, erythritol, glyceryl palmitostearate, tributyl citrate, triethyl citrate, glyceryl monostearate, glycerin monostearate, gelatin, hydrogenated soybean oil, glyceryl monostearate, Hydrogenated oil, carboxymethyl cellulose, sodium carboxymethyl cellulose, sodium carboxymethyl starch, crosscarmellose sodium, aminoalkyl methacrylate copolymer E, low-substituted hydroxypropyl ether of cellulose, polyvinyl pyrollidone,polyethylene glycol, cellulose trimellitate, hydroxymethyl cellulose acetate succinate, resins like shellac, methylmethacrylate-methacrylic acid, methacrylic acid-ethyl acrylate, zein, hydroxyethyl-ethylcellulose phthalate, cellulose aceto succinate, polylactic acid, polyglycolic acid, gelatinized starch, ammonium hydroxide, sodium hydroxide, potassium hydroxide, magnesium hydroxide, calcium hydroxide, gums, liposomes, cyclodextrins, chitosan, gliadin, hordein, prolamine, sucrose, sorbitol, glycerin,triethylamine, diethylamine, trimethylamine, methylamine, dimethylamine, isopropylamine, triethanolamine, diethanolamine, disodium monohydrogen phosphate, dipotassium monohydrogen phosphate, mixtures thereof and the like.

Methacrylate-based coatings are purchased under the trade name Eudragit®. Many varieties of Eudragit® coatings are available. Water insoluble Eudragit® E PO, for instance, can be preferred. EUDRAGIT® E PO is the powder form of cationic copolymer based on dimethylaminoethyl methacrylate, butyl methacrylate, and methyl methacrylate.

The taste masking layer further includes a pore former or mixtures of pore formers and a surfactant or mixtures of surfactants.

Pore formers are, but not limited to, polyethylene glycol, polyvinylpyrrolidone, dextrose, mannose, fructose, sucrose, glucodifructose, calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, mixtures thereof and the like. Using of pore formers and surfactants are critical for adjusting of flurbiprofen dissolution profile besides taste masking properties.

Surfactants are, but not limited to, alpha tocopherol, cetostearyl alcohol, cetrimide, cetylpyridinium chloride, sodium methyl lauroyl taurate, sodium methyl stearoyl taurate, polyoxyethylene stearate, macrogol 15 hydroxystearate, sodium methyl palmitoyl taurate, ammonium lauryl sulphate, ammonium laureth sulphate, sodium cocoyl sarcosinate, triethanolamine lauryl sulphate, triethanolamine laureth sulphate, disodium oleamide sulfosuccinate, disodium laureth sulfosuccinate, disodium dioctyl sulfosuccinate, polysorbate 80, sodium lauryl sulfate, sugar esters of fatty acids, poloxamer, docusate sodium, polyoxyethylene sorbitan fatty acid esters, polyoxyethylene glycol sorbitan fatty acid esters, non hydrogentated polyoxyethylene castor oil derivatives, cetyltrimethyl ammonium bromide, benzalkonium chloride, benzethonium chloride, alkyidimethylbenzylammonium salt, tetrabutyl ammonium bromide, alkylammonium salts, fusidic acid salts, fatty acid derivatives of amino acids, oligopeptides, polypeptides, glyceride derivatives of amino acids, lecithins, hydrogenated lecithins, lysolecithins or hydrogenated lysolecithins, phospholipids or derivatives, lysophospholipids or derivatives, carnitine fatty acid ester salts, salts of alkylsulfates; fatty acid salts, sodium docusate, acyl lactylates, mono- or di-acetylated tartaric acid esters of mono- or di-glycerides, succinylated mono- or di-glycerides, citric acid esters of mono- or di-glycerides, fatty alcohols, glycerol fatty acid esters, acetylated glycerol fatty acid esters, lower alcohol fatty acids esters, propylene glycol fatty acid esters, sorbitan fatty acid esters, polyethylene glycol sorbitan fatty acid esters, sterols and sterol derivatives, polyoxyethylated sterols or sterol derivatives, polyethylene glycol alkyl ethers, sugar esters, sugar ethers, lactic acid derivatives of mono- or di-glycerides, oil-soluble vitamins/vitamin derivatives, PEG fatty acid esters, polyglycerized fatty acid, polyoxyethylene-polyoxypropylene block copolymers, transesterification products of a polyol with at least one member of the group consisting of glycerides, vegetable oils, hydrogenated vegetable oils, corn oil, olive oil, peanut oil, palm kernel oil, almond oil, glycerol, propylene glycol, ethylene glycol, polyethylene glycol, sorbitol, pentaerythritol, sodium cocoyl isethionate, sodium lauroyl sarcosinate, sodium methyl cocoyl taurate, sodium methyl lauroyl taurate, sodium methyl stearoyl taurate, sodium methyl palmitoyl taurate, ammonium lauryl sulphate, alkaryl sulphonates, alkyl succinates, alkyl sulphosuccinates, N-alkoyl sarcosonates, alkyl phosphates, alkyl ether phosphates, alpha-olefm sulphonates, acyl methyl taurates and the like.

The term "surfactant" embraces co-surfactants. Co-surfactants, such as glyceryl monooleate, polyethylenglycol monocaprylate, glyceryl mono- dicaprylate, glyceryl mono-dicapryl, can be used alone or with surfactants.

The taste-masking layer (as described herein) can be applied to the flurbiprofen particles by any suitable method, for example fluidized bed coating methods.

Taste masking coating solution can be prepared as following manner;

**Table 1**

| Ingredients | % (w/w) |
|---|---|
| Sodium dodecyl sulphate | 2,16 |
| Eudragit E PO | 2,27 |
| Triethyl citrate | 0.23 |
| 0.1 N HCL | 23.29 |
| Deionized water | 72,05 |
| **Total** | **100** |

Taste masking coating solution comprising ; (i) an inorganic acid or mixtures of inorganic acids or, (ii) an organic acid or mixtures of organic acids or, (iii) mixtures of organic acids and inorganic acids or, (iv) mixture of an organic acid and an inorganic acid or, (v) an organic solvent or mixtures of organic solvents or, (vi) mixtures of one or more organic solvents and any alternative (i) to (v) or, (vii) de-ionized water and any alternative (i) to (v) or, (viii) de-ionized water and, (ix) a pore former or mixtures of pore formers and, (x) a coating agent or mixtures of coating agents and, (xi) a surfactant or mixtures of surfactant, x) optionally and additionally an excipient or mixtures of excipients.

Taste masking coating solution may include further excipients .

Organic solvents, used in preparation of solution are, but not limited to, alcohols such as methanol, methanol, ethanol, propyl alcohol, isopropyl alcohol, 2-methyl-1-propanol etc.; ketones such as acetone, methyl ethyl ketone, methyl isobutyl ketone etc.; esters such as methyl acetate, ethyl acetate, propyl acetate, isopropyl acetate, ethyl formate etc.; hydrocarbons such as heptane; halogenated hydrocarbons such as dichloromethane.

Preferable examples of organic acids are formic acid, acetic acid, fumaric acid, tartaric acid, maleic acid, citric acid, succinic acid, malic acid, methanesulfonic acid, p-toluenesulfonic acid.

Preferable example of inorganic acid is hydrochloric acid which is in the form of solution in water.

This invention also provides a preparation method which has following steps : a) Flurbiprofen is granulated with the binder solution in a suitable granulator such as high shear mixer granulator, b) Flurbiprofen granules, obtained from step a, are dried in a suitable dryer such as fluid bed dryer, c) Dried granules, obtained from step b, are sieved from frewit sieve, d) Sieved granules, obtained from step c, are loaded to suitable granulator for coating process, e) Loaded particles in step d are coated with taste masking film (solution), f) Coated particles, obtained from step e, are tabletted.

## Claims

1. A fast disintegrating or fast dissolving dosage form of flurbiprofen, comprising:
- an effective amount of flurbiprofen or pharmaceutically acceptable salts thereof and - at least one pharmaceutically acceptable excipient **characterized in that** flurbiprofen particles are coated with a mixture comprising; a. coating agent or mixtures of coating agents and b. a pore former or mixtures of pore formers and c. a surfactant or mixtures of surfactants.

2. According to claim 1, the fast disintegrating or fast dissolving dosage form of flurbiprofen is disintegrated in oral cavity in less than three minutes.

3. According to claim 2, the fast disintegrating or fast dissolving dosage form of flurbiprofen is disintegrated in oral cavity in less than two minutes.

4. According to claim 3, the fast disintegrating or fast dissolving dosage form of flurbiprofen is disintegrated in oral cavity in less than one minutes.

5. According to claim 1, coating agent is selected from the group consisting of ethyl cellulose, methyl cellulose, hydroxypropyl-methyl cellulose, hydroxyethyl cellulose, hydroxypropyl cellulose,polyvinylalcohol, polyvinyl acetate , cellulose acetate ,cellulose acetate phthalate, methylcellulose phthalate, hydroxymethyl cellulose phthalate, hydroxypropylmethylcellulose phthalate, hydroxypropylmethylcellulose succinate, cellulose acetate butyrate, methacrylate copolymers ,polymethacrylates, acetyltributyl citrate, erythritol, glyceryl palmitostearate, tributyl citrate, triethyl citrate, glyceryl monostearate, glycerin monostearate, gelatin, hydrogenated soybean oil, glyceryl monostearate,Hydrogenated oil, carboxymethyl cellulose,sodium carboxymethyl cellulose, sodium carboxymethyl starch, crosscarmellose sodium, aminoalkyl methacrylate copolymer E, low-substituted hydroxypropyl ether of cellulose,polyvinyl pyrollidone,polyethylene glycol,cellulose trimellitate,hydroxymethyl cellulose acetate succinate, resins like shellac, methylmethacrylate-methacrylic acid , methacrylic acid-ethyl acrylate,zein, hydroxyethyl-ethylcellulose phthalate, cellulose aceto succinate, polylactic acid,polyglycolic acid, gelatinized starch, ammonium hydroxide,sodium hydroxide, potassium hydroxide, magnesium hydroxide, calcium hydroxide, gums, liposomes, cyclodextrins, chitosan, gliadin,hordein,prolamine,sucrose, sorbitol, glycerin,triethylamine, diethylamine, trimethylamine, methylamine, dimethylamine, isopropylamine, triethanolamine, diethanolamine, disodium monohydrogen phosphate, dipotassium monohydrogen phosphate, mixtures thereof.

6. According to claim 1, pore former is selected from the group consisting of polyethylene glycol, polyvinylpyrrolidone ,dextrose, mannose, fructose,sucrose, glucodifructose,calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, mixtures thereof.

7. According to claim 1, surfactant is selected from the group consisting of alpha tocopherol,cetostearyl alcohol,cetrimide,cetylpyridinium chloride,sodium methyl lauroyl taurate, sodium methyl stearoyl taurate ,polyoxyethylene stearate,macrogol 15 hydroxystearate,sodium methyl palmitoyl taurate, ammonium lauryl sulphate, ammonium laureth sulphate, sodium cocoyl sarcosinate, triethanolamine lauryl sulphate, triethanolamine laureth sulphate, disodium oleamide sulfosuccinate, disodium laureth sulfosuccinate, disodium dioctyl sulfosuccinate,polysorbate 80, sodium lauryl sulfate, sugar esters of fatty acids, poloxamer, docusate sodium, polyoxyethylene sorbitan fatty acid esters, polyoxyethylene glycol sorbitan fatty acid esters, non hydrogentated polyoxyethylene castor oil derivatives,cetyltrimethyl ammonium bromide, benzalkonium chloride, benzethonium chloride, alkyidimethylbenzylammonium salt, tetrabutyl ammonium bromide, alkylammonium salts, fusidic acid salts, fatty acid derivatives of amino acids, oligopeptides, polypeptides, glyceride derivatives of amino acids, lecithins, hydrogenated lecithins, lysolecithins or hydrogenated lysolecithins, phospholipids or derivatives, lysophospholipids or derivatives ,carnitine fatty acid ester salts, salts of alkylsulfates; fatty acid salts, sodium docusate, acyl lactylates, mono- or di-acetylated tartaric acid esters of mono- or di-glycerides, succinylated mono- or di-glycerides, citric acid esters of mono- or di-glycerides, fatty alcohols, glycerol fatty acid esters, acetylated glycerol fatty acid esters, lower alcohol fatty acids esters, propylene glycol fatty acid esters, sorbitan fatty acid esters, polyethylene glycol sorbitan fatty acid esters, sterols and sterol derivatives, polyoxyethylated sterols or sterol derivatives, polyethylene glycol alkyl ethers, sugar esters, sugar ethers, lactic acid derivatives of mono- or di- glycerides, oil-soluble vitamins/vitamin derivatives, PEG fatty acid esters, polyglycerized fatty acid, polyoxyethylene-polyoxypropylene block copolymers, transesterification products of a polyol with at least one member of the group consisting of glycerides, vegetable oils, hydrogenated vegetable oils, corn oil, olive oil, peanut oil, palm kernel oil, almond oil , glycerol, propylene glycol, ethylene glycol, polyethylene glycol, sorbitol ,pentaerythritol, sodium cocoyl isethionate, sodium lauroyl sarcosinate, sodium methyl cocoyl taurate, sodium methyl lauroyl taurate, sodium methyl stearoyl taurate, sodium methyl palmitoyl taurate , ammonium lauryl sulphate, alkaryl sulphonates, alkyl succinates, alkyl sulphosuccinates, N-alkoyl sarcosonates, alkyl phosphates, alkyl ether phosphates, alpha-olefm sulphonates , acyl methyl taurates or mixtures thereof.

8. A preparation method of flurbiprofen or pharmaceutically acceptable salts thereof fast dissolving or fast disintegrating tablet **characterized in that** said method has the following steps : a) Flurbiprofen is granulated with the binder solution in a granulator and b) Flurbiprofen granules, obtained from step a, are dried in a suitable dryer and c) Dried granules, obtained from step b, are sieved from a sieve, d) Sieved granules, obtained from step c, are loaded to a granulator for coating process, e) Loaded particles in step d are coated with taste masking film , f) Coated particles, obtained from step e, are tabletted.

9. Taste masking coating solution to the flurbiprofen or pharmaceutically acceptable salts thereof fast dissolving or fast disintegrating tablet comprising ;
(i) an inorganic acid or mixtures of inorganic acids or, (ii) an organic acid or mixtures of organic acids or, (iii) mixtures of organic acids and inorganic acids or, (iv) mixture of an organic acid and an inorganic acid or (v) an organic solvent or mixtures of organic solvents or (vi) mixtures of one or more organic solvents and any alternative (i) to (v) or (vii) de-ionized water and any alternative (i) to (v) or (viii) de-ionized water and (ix) a pore former or mixtures of pore formers and (x) a coating agent or mixtures of coating agents and (xi) a surfactant or mixtures of surfactants xii)optionally and additionally an excipient or mixtures of excipients.

10. Fast disintegrating or fast dissolving dosage formulation of flurbiprofen or pharmaceutically acceptable salts thereof comprising; flurbiprofen is in the range of from 10 % to 35 %, binder is in the range of from 0.1 % to 5 %, coating agent is in the range of from 0.1 % to 5 %, pore former is in the range of from 0.01 % to 5 %, disintegrant is in the range of from 20 % to 90 %, sweetener is in the range of from 0.1 % to 5 %, stabilizing agent is in the range of from 0.1 % to 5 %, flavour is in the range of from 0.1 % to 5 %, glidant is in the range of from 0.1 % to 5 %, lubricant is in the range of from 0.1 % to 20 %, surfactant is in the range of from 0.1 % to 10 %, by weight .

11. According to preceding claims, the fast disintegrating or fast dissolving dosage form is orally disintegrating tablet.
